# FASCICULE DE BREVET EUROPEEN

(11) **EP 2 358 391 B1**
(45) Date de publication et mention de la délivrance du brevet: **10.12.2014**
(21) Numéro de dépôt: 09803869.8
(22) Date de dépôt: 16.12.2009
(51) Int. Cl.: A61K 39/395, A61P 43/00

(54) **UTILISATION D'UN CONCENTRE D'IMMUNOGLOBULINES G (IGG) APPAUVRI EN ANTICORPS ANTI-A ET ANTI-B POUR LE TRAITEMENT DE L'ICTERE NEONATAL PAR INCOMPATIBILITE FOETOMATERNELLE DANS LE SYSTEME ABO**
VERWENDUNG EINES ANTI-A- UND ANTI-B-ANTIKÖRPER-ARMEN IMMUNGLOBULIN G (IGG)-KONZENTRATS ZUR BEHANDLUNG VON GELBSUCHT BEI SÄUGLINGEN DURCH MUTTER-KIND-INKOMPATIBILITÄT DES AB0-SYSTEMS
USE OF AN IMMUNOGLOBULIN G (IGG) CONCENTRATE DEPLETED OF ANTI-A AND ANTI-B ANTIBODIES FOR TREATING NEONATAL JAUNDICE CAUSED BY MATERNAL-FOETAL INCOMPATIBILITY WITH RESPECT TO THE ABO SYSTEM

(30) Priorité: 17.12.2008 FR 0807105
(43) Date de publication de la demande: 24.08.2011
(73) Titulaire: Laboratoire Français du Fractionnement et des Biotechnologies, 91940 Les Ulis (FR)
(72) Inventeur: ELZAABI, Mazen, 75012 Paris (FR)
(74) Mandataire: Novagraaf Technologies
(86) Numéro de dépôt international: PCT/FR2009/052558
(87) Numéro de publication internationale: WO 2010/076496

(56) Documents cités:
- FR-A- 2 895 263
- TANYER G ET AL: "Multiple dose IVIG treatment in neonatal immune hemolytic jaundice." JOURNAL OF TROPICAL PEDIATRICS FEB 2001, vol. 47, no. 1, février 2001 (2001-02), pages 50-53, XP008109562 ISSN: 0142-6338
- HAMMERMAN C ET AL: "Intravenous immune globulin in neonatal ABO isoimmunization: factors associated with clinical efficacy." BIOLOGY OF THE NEONATE 1996, vol. 70, no. 2, 1996, pages 69-74, XP008109561 ISSN: 0006-3126
- ERGAZ Z ET AL: "Carboxyhemoglobin levels in neonatal immune hemolytic jaundice treated with intravenous gammaglobulin." VOX SANGUINIS 1995, vol. 69, no. 2, 1995, pages 95-99, XP008109565 ISSN: 0042-9007
- DATABASE MEDLINE [Online] US NATIONAL LIBRARY OF MEDICINE (NLM), BETHESDA, MD, US; septembre 2006 (2006-09), HUANG WEI-MIN ET AL: "[Clinical study of early interventions for ABO hemolytic disease of the newborn]" XP002540810 Database accession no. NLM16982453 & NAN FANG YI KE DA XUE XUE BAO = JOURNAL OF SOUTHERN MEDICAL UNIVERSITY SEP 2006, vol. 26, no. 9, septembre 2006 (2006-09), pages 1350-1351 , 13, ISSN: 1673-4254
- KNEZEVIC-MARAMICA I ET AL: "Intravenous immune globulins: an update for clinicians" TRANSFUSION, AMERICAN ASSOCIATION OF BLOOD BANKS, BETHESDA, MD, US, vol. 43, no. 10, 1 octobre 2003 (2003-10-01), pages 1460-1480, XP002400780 ISSN: 0041-1132

## Description

### Domaine technique

La présente invention se rapporte à l'utilisation d'un concentré d'immunoglobulines G (IgG) appauvri en anticorps anti-A (AcaA) et anti-B (AcaB) pour la fabrication d'un medicament destiné au traitement de l'ictère néonatal par incompatibilité fcetomaternelle dans le système ABO.

Dans la description ci-dessous, les références entre crochets ([ ]) renvoient à la liste des références présentée après les exemples.

### Etat de la technique

La maladie hémolytique du nouveau-né (MHN) est une affection du foetus ou du nouveau-né due à une incompatibilité entre les anticorps anti-érythrocytaires de la mère et les hématies de l'enfant. Les anticorps anti-érythrocytaires entraînent une hémolyse survenant déjà in utero et, selon la gravité du tableau clinique, une anémie parfois sévère chez l'enfant. La bilirubine étant le produit de dégradation de l'hème (constituant de l'hémoglobine) transporté dans le sang, le tableau peut aller d'une hyperbilirubinémie s'accompagnant d'un ictère chez l'enfant, jusqu'à une pathologie très lourde avec anasarque (hydrops foetalis) et accouchement d'un enfant mort-né. Non traitée, l'hyperbilirubinémie peut induire un ictère nucléaire chez le nouveau-né.

La connaissance du tableau clinique et de son exploration diagnostique est d'une extrême importance, d'autant plus que l'on dispose aujourd'hui de moyens efficaces pour prévenir la MHN (prophylaxie anti-D ou facteur Rhésus) ainsi que de traitements efficaces (exsanguino-transfusion intra-utérine / postnatale, thérapie néonatale aux UV).

Néanmoins, tout cela n'est possible que si le risque est reconnu à temps, c'est-à-dire avant la grossesse (notamment par détermination du groupe sanguin de la mère et éventuellement du père), en début de grossesse (éventuellement confirmation des groupes sanguins et recherche d'alloanticorps, y compris d'anticorps immuns anti-A / anti-B) ou au cours de la grossesse (contrôle de l'évolution du titre toutes les 3 à 4 semaines). En outre, la détermination du groupe sanguin du père de l'enfant peut être très utile (phénotype avec indices permettant de conclure au génotype Rhésus probable), car il est alors possible d'en déduire le groupe sanguin probable de l'enfant.

Le véritable risque de MHN peut être estimé d'après le groupe sanguin de la mère ou le faible titre d'un alloanticorps et du groupe sanguin présumé de l'enfant, établi à partir du groupe sanguin du père. Si la mère fait partie d'un groupe sanguin à risque (p.ex. O ou Rhésus D négatif), il ne faut en aucun cas renoncer aux contrôles de l'évolution du titre.

Dans de rares cas, lors de constellation de risques analogues, la détermination biomoléculaire du groupe sanguin de l'enfant à partir d'une biopsie choriale peut être indiquée.

L'incompatibilité ABO survient typiquement dans les cas d'une mère de groupe sanguin O et d'un enfant de groupe sanguin A ou B.

Les anticorps anti-ABO de l'isotype IgM, présents à l'état naturel, ne traversent pas la barrière placentaire. Bien que le système ABO ne soit pas encore complètement développé et exprimé à la naissance - la détermination définitive des groupes sanguins ne devrait pas être effectuée avant l'âge de 6 mois chez l'enfant - le groupe A ou B peut être décelé sur les érythrocytes du foetus à un stade précoce de la grossesse. C'est pourquoi l'immunisation de la mère est relativement fréquente même dans cette constellation. D'autre part, des anticorps immuns IgG anti-A et anti-B peuvent être induits par des protéines étrangères, indépendamment d'une grossesse ou d'une transfusion sanguine antérieure. Cependant, les anticorps IgG anti-A ou anti-B ont presque toujours un plus faible pouvoir hémolytique que les anticorps analogues en cas d'incompatibilité Rhésus. Par conséquent, l'évolution de la MHN est plus discrète.

L'ictère apparaît dans 70 % des cas entre 24 et 72 heures après la naissance. Il est dans tous les cas dépisté par voie visuelle et instrumentale, souvent avant la réception du résultat du sang du cordon, motivant la mise en route avec succès de la photothérapie. En aucun cas, la connaissance précoce des résultats du groupe ne débouche sur une mesure thérapeutique, préventive ou curative tant que le diagnostic d'ictère n'a pas été posé. Elle engendre tout au plus une vigilance accrue face à la coloration du nouveau-né.

L'ictère est de très loin le plus fréquent des symptômes observés à la période néonatale.

La première caractéristique est que, contrairement à l'adulte, l'ictère du nouveau-né est, dans l'immense majorité des cas, à bilirubine (BR) indirecte.

Ce pigment, en s'accumulant dans l'organisme va intéresser tous les organes, mais surtout le foie (qui, avant tout, rend compte de cette accumulation), le sang (qui véhicule et stocke en partie le pigment), la peau et le cerveau, avec un risque potentiel constant d'encéphalopathie bilirubinique, qui justifie la plus grande rigueur dans la conduite du diagnostic et du traitement.

Traditionnellement, les enfants sont traités au moyen d'immunogoblulines intraveineuses (IGIV) et d'une photothérapie, et dans les cas les plus graves, par transfusion d'échange.

Des etudes ont porté sur le traitement des nouveau-nés atteints de la maladie hémolytique à incompatibilité Rh et/ou ABO au moyen de fortes doses d'IGIV (immunoglobulines intraveineuses), et ont montré que l'administration d'IGIV réduisait le nombre d'enfants ayant besoin de transfusions d'échanges, ainsi que la durée du traitement par photothérapie (Gottstein et al. (2003), Archives of Disease in Childhood: Fetal and Neonatal Edition; vol. 88, no. 1, p. F6-F10 [2]).

Il a également été montré que la thérapie des nouveau-nés atteints de la maladie hémolytique à incompatibilité Rh et/ou ABO au moyen de fortes doses d'IGIV réduit l'hémolyse, les niveaux de bilirubine du sérum et la nécessité de pratiquer des transfusion d'échange (Alplay et al. (1999), Acta Paediatrica, International Journal of Paediatrics; vol. 88, no. 2, p. 216-219 [3]).

Toutefois, une étude a montré que l'administration d'IGIV à des nouveau-nés atteints d'une jaunice hémolytique isoimmune due à des incompatibilités Rh et ABO engendrait des resultats significativement meilleurs pour le traitement de l'isoimmunisation Rh par rapport à celui de l'isoimmunisation ABO. En effet, les besoins en transfusion d'échange des nouveau-nés présentant une hyperbilirubinémie due à une incompatibilité Rh étaient diminués à l'issue d'un traitement par administration d'IGIV par rapport à un traitement par photothérapie. Par contre, dans le cas des nouveau-nés présentant une incompatibilité ABO, la photothérapie et l'administration d'IVIG n'ont montré aucune différence en terme de résultats (Nasseri et al. (2006) Saudi Med J. ; 27(12) : 1827-30 [4]).

Le document Tanyer G. et al. (« Multiple dose IVIG treatment in neonatal immune hemolytic jaundice ». Journal of tropical pediatrics Feb 2001, vol. 47, no. 1, pages 50-53) décrit l'utilisation d'IVIG dans le traitement de la maladie hémolytique isoimmune due à une incompatibilité ABO. Toutefois, ce document ne décrit aucune caractéristique des IGIV utilisées, ni aucune méthode de préparation susceptible d'être mise en oeuvre pour les obtenir. Tous les groupes de patients traités ont reçu un traitement complémentaire par photothérapie avant traitement par les concentrés d'IGIV, ce traitement ayantpermis la diminution du taux de bilirubine pour atteindre la limite de sécurité. Ainsi, l'effet thérapeutique est en parti dû à cette photothérapie. De plus, le traitement par simple dose administré est un échec, comme le montre la grande variabilité de la réponse obtenue. Or, le traitement par doses multiples implique les mêmes IGIV que celui par dose unique. L'amélioration de la réponse observée dans le cas d'une administration de doses multiples est donc uniquement due à la posologie d'administration, et non à une quelconque caractéristique technique des IGIV en tant que tel. Enfin, les groupes testés ont quand même dû subir des échanges transfusionnels, ce qui démontre que ce traitement n'est pas un « succès thérapeutique ».

De plus, l'administration de fortes doses d'IGIV doit être considérée comme un traitement à risque chez certains patients présentant une incompatibilité ABO. En effet, les IGIV sont des concentrés d'immunoglobulines issues du plasma humain, et comportent à ce titre des anticorps anti-A et des anticorps anti-B.

De nombreuses publications scientifiques indiquent que l'injection d'immunoglobulines G (IgG) obtenues par les techniques de fractionnement classiques, comme la précipitation à l'éthanol, ou la précipitation par l'acide octanoïque (Steinbuch et al. (1969) Rev. Franc. Et. Clin, et Biol., XIV, 1054 [5]), peuvent provoquer des hémolyses accidentelles, dont certaines sévères, chez des patients en traitement. On peut à titre d'exemple citer les publications de Buchta C. et al, Biologicals, 33, 2005, 41- 48 ([6]), Wilson J. R. et al, Muscle & Nerve, 29(9), 1997, 1142- 1145 ([7]), Copelan E.A et al, Transfusion, 26, 1986, 410-412 ([8]) et Misbah S.A et al, Drug Safety, 9, 1993, 254-262 ([9]). L'étude des effets de ces IgG sur le sang des patients présentant une hémolyse, mise en oeuvre notamment par le test de Coombs direct (DCT), a montré que les hématies sont couvertes d'immunoglobulines dirigées contre les antigènes A, B ou D présents à leur surface, provoquant ainsi leur hémolyse.

C'est pourquoi les IgG actuellement disponibles dans le commerce sont obtenues à partir de plasmas sélectionnés pour éviter la présence de forts titres en anti-A ou anti-B.

Selon la Pharmacopée Européenne (Méthode 2.6.20 également appelée test de Coombs indirect (ICT), 1997), les IGIV ne doivent pas présenter d'agglutination des globules rouges A ou B au test de Coombs indirect (ICT) in vitro à la dilution 1/64 mis en oeuvre avec une solution d'IgG de concentration initiale ramenée à 30 g/l. En d'autres termes, le titre maximal autorisé par la pharmacopée européenne doit être inférieur à 64 (en inverse de dillution), conformément à la méthode 2.6.20 de la pharmacopée européenne, c'est-à-dire qu'une composition d'IGIV diluée au 1/64 ne doit pas provoquer d'agglutination des globules rouges (hématies).

Des résultats négatifs au test ICT, c'est-à-dire une absence d'agglutination des hématies, en présence de solutions d'IGIV dont les dilutions sont inférieures à la dilution 1/64, selon la Pharmacopée Européenne, démontrent un faible taux en anticorps anti-A et anti-B acceptés par celle-ci. Toutefois, même avec des concentrés d'IgG donnant un résultat négatif au test prescrit par la Pharmacopée Européenne, c'est-à-dire ceux dont la dilution est inférieure à 1:64, les risques de réactions hémolytiques ne peuvent être exclus ([6]).

Par ailleurs, les Pharmacopées américaine et japonaise ne contiennent aucune disposition sur la nécessité de contrôler les teneurs résiduelles en anticorps anti-A et anti-B.

Les anticorps anti-A et anti-B sont partiellement éliminés au cours de la préparation de concentrés d'IgG par les procédés classiques, comme le fractionnement éthanolique, mais on en observe une teneur résiduelle qui peut être supérieure à la limite des normes hautes de la Pharmacopée Européenne. De plus, les concentrés préparés selon le procédé mis au point par la Demanderesse et décrit dans sa demande de brevet WO 02/092632 en contiennent davantage que ceux obtenus par le fractionnement à l'éthanol. Certains lots de concentrés d'IgG peuvent présenter des teneurs supérieures au seuil admis par la Pharmacopée Européenne pour chacun d'entre eux.

Par conséquent, il apparaît nécessaire de disposer d'un traitement de l'ictère nénonatal par incompatibilité foetomaternelle dans le système ABO, qui puisse être administré aux nouveau-nés souffrant d'une incompatibilité ABO, sans crainte de provoquer une hémolyse supplémentaire.

### Description de l'invention

C'est dans ce but et au cours de ses importantes recherches que la Demanderesse a mis en évidence de manière surprenante qu'une composition d'immunoglobulines G présentant des titres respectifs en anticorps anti-A et anti-B conformes à un résultat négatif au test de Coombs indirect in vitro peut être utilisée pour le traitement de l'ictère nénonatal par incompatibilité foetomaternelle dans le système ABO ou de la maladie hémolytique du nouveau-né par incompatibilité ABO, sans induire les effets secondaires indésirables constatés avec les compositions de l'art antérieur.

Ainsi, l'invention concerne une composition d'Immunoglobulines G (IgG) à usage thérapeutique, comprenant des titres respectifs en anticorps anti-A et anti-B conformes à un résultat négatif au test de Coombs indirect (Méthode 2.6.20 de la Pharmacopée européenne), **pour son utilisation dans** le traitement de l'ictère nénonatal par incompatibilité foetomaternelle dans le système ABO ou de la maladie hémolytique du nouveau-né par incompatibilité ABO.

On entend par « immunoglobulines G », au sens de la présente invention, des IgG polyclonales, qui peuvent être obtenues à partir du plasma sanguin ou d'une fraction de plasma sanguin déjà enrichie en IgG. Les compositions, ou concentrés d'IgG à usage thérapeutique présentent avantageusement des concentrations en IgG communément mises en oeuvre, comprises de préférence entre 50 g/l et 100 g/l.

On entend par « usage thérapeutique », au sens de la présente invention, une utilisation visant une amélioration de l'état de santé du patient, par exemple la diminution de la virulence de l'ictère, ou encore sa disparition totale ou partielle, voire la guérison du patient.

On entend par « titres respectifs en anticorps anti-A et anti-B », au sens de la présente invention, le titre tel que définit dans la Pharmacopée européenne (Pharmacopée européenne 2.6.20) et mesuré par le test de Coombs indirect (test ICT). En d'autres termes, le titre est la dilution à partir de laquelle une agglutination est détectée conformément à la Méthode 2.6.20 de la pharmacopée européenne. Encore en d'autres termes, le titre est la dilution à partir de laquelle une hémolyse est observée.

Le test ICT (Méthode 2.6.20 de la pharmacopéen européenne) consiste à une suspension d'hématies mises en contact avec la composition d'IgG **utilisée pour** l'invention, une solution d'anticorps (antiglobulines) dirigés contre des motifs de l'IgG humaine. Ces anticorps se fixent sur des anticorps anti-A ou anti-B attachés aux hématies et permettent ainsi leur agglutination par formation de ponts entre les IgG. L'essai de recherche d'anticorps anti-A ou anti-B s'inspire directement de ce test classique en sérologie hématologique (test de Coombs indirect).

Dans le cadre de l'invention, la Méthode 2.6.20 de la Pharmacopée européenne peut être mise en oeuvre de la manière suivante : on prépare 2 séries identiques de dilutions de la composition d'IgG **utilisée pour** l'invention dans une solution de *chlorure de sodium R* à 9 g/l. A chaque dilution de la première série, on ajoute un volume égal d'une suspension à 5 % VN de cellules rouges A lavées 3 fois au préalable avec la solution de chlorure de sodium. A chaque dilution de la deuxième série, on ajoute un volume égal d'une suspension à 5 % V/V de cellules rouges B lavées 3 fois au préalable avec la solution de chlorure de sodium. On laisse incuber la suspension à 37°C pendant 30 min, puis on lave les cellules avec la solution de chlorure de sodium. On met chaque suspension en contact avec un réactif antiglobuline humaine polyvalent pendant 30 min. Sans centrifuger les mélanges, on recherche par examen microscopique une éventuelle agglutination. Si la composition d'IgG avant dilution a une teneur en immunoglobulines supérieure à 30 g/l, une dilution pour atteindre cette concentration de 30 g/l doit être effectuée avant de préparer les dilutions à utiliser dans l'essai. Les dilutions au 1/64 ne présentent pas de signes d'agglutination.

Lorsque le taux d'anticorps anti-A et anti-B est très faible dans les concentrés d'IgG, le test ICT est négatif, a fortiori dans les conditions de la Pharmacopée Européenne, étant donné que les réactions d'agglutination des hématies n'ont plus lieu, même avec l'ajout d'anticorps anti-IgG humaines, car la densité d'anticorps anti-A et anti-B est trop faible pour l'établissement de pontages entre les hématies par des liaisons anticorps anti-A et anti-B fixés sur les hématies et les anticorps anti-IgG humaines.

Le test de la Pharmacopée Européenne 2.6.20, ou test ICT, est le seul test reconnu au niveau réglementaire, et toutes les IGIV commercialisées en Europe doivent être conformes à ce test, c'est-à-dire présenter une absence d'agglutination à une dilution au 1/64. Le titre maximal autorisé par la pharmacopée doit donc être (en inverse de dillution) inférieur à 64 (< 64). C'est-à-dire qu'à une dilution au 1/64, le produit testé n'induit pas d'agglutination, conformément à ce qui est décrit dans la Méthode 2.6.20 de la pharmacopéen européenne.

On entend par « résultat négatif au test de Coombs indirect », au sens de la présente invention, une absence d'agglutination des hématies, mesurée conformément à la Méthode 2.6.20 de la Pharmacopée européenne, lors de leur mise en contact avec les IgG de la composition **utilisée pour** l'invention, en présence d'antiglobuline humaine polyvalente.

La composition d'immunoglobulines G **utilisée pour** l'invention peut donc présenter, de manière avantageuse, des titres respectifs en anticorps anti-A et anti-B conformes à un résultat négatif au test de Coombs indirect in vitro de 64 en inverse de dilution, c'est-à-dire négatifs à la dilution 1/64.

La composition d'immunoglobulines G **utilisée pour** l'invention présente avantageusement un titre (en inverse de dillution) compris entre 0, 0 étant avantageusement exclu, et 8. A ces titres en anticorps anti-A et anti-B, la composition d'IgG **utilisée pour** l'invention présente un résultat conforme à l'ICT, c'est-à-dire une absence d'agglutination. C'est-à-dire qu'à une dilution comprise entre 0 (aucune dilution), 0 étant avantageusement exclu, et 1/8, la composition d'IgG **utilisée pour** l'invention n'induit pas d'agglutination, conformément à ce qui est décrit dans la Méthode 2.6.20 de la pharmacopéen européenne. Ainsi, un titre de 8 signifie qu'une agglutination est observée au-delà de la 8^{ème} dilution de l'échantillon, (dilution au 1/8). Un titre de 0 signifie qu'aucune agglutination n'est détectée, même lorsque l'échantillon n'est pas dilué.

Avantageusement, le titre en anticorps anti-A et en anticorps anti-B, donnés en inverse de dilution, est inférieur à 4, ou inférieur à 2. De telles compositions d'IgG ne provoquent pas d'agglutination lorsqu'elles sont diluées respectivement 4 fois (dilution au ¼) ou 2 fois (dilution au ½).

Ainsi, les compositions (ou concentrés) d'IgG permettant de réaliser l'invention présentent des titres en anticorps anti-A et anti-B nettement inférieures à ceux observés dans les concentrés d'IgG standards, c'est-à-dire à ceux obtenus par fractionnement éthanolique et/ou par la mise en oeuvre des techniques de purification associant des chromatographies sans étape complémentaire d'élimination des anticorps considérés.

De plus, les titres sont nettement inférieurs aux seuils acceptés par la Pharmacopée Européenne, ce qui limite très significativement les risques d'hémolyse chez certains receveurs en traitement. La mise en oeuvre du test de Coombs indirect, avec des compositions d'IgG utilisées pour l'invention, peut conduire à des résultats négatifs, même avec des échantillons d'IgG en tant que tels, non dilués.

Les concentrés d'IgG **utilisés pour** l'invention sont donc définis par une absence notable des principes actifs anticorps anti-A et anti-B qui sont dirigés contre les épitopes présents sur les globules rouges.

La Demanderesse a trouvé qu'une composition d'IgG telle que décrite est particulièrement adaptée au traitement de l'ictère nénonatal par incompatibilité foetomaternelle dans le système ABO.

Avantageusement, la composition d'immunoglobulines G **utiliséee pour** l'invention peut donc présenter, de manière avantageuse, des titres respectifs en anticorps anti-A et anti-B égaux à 64 en inverse de dilution, ou compris entre 0 et 8, ou inférieurs à 4, par exemple inférieurs à 2. Avantageusement, dans ces cas de figure, le test de Coombs est mis en oeuvre avec une solution d'IgG de concentration intitiale ramenée à 30 g/l.

On entend par « ictère nénonatal par incompatibilité foetomaternelle dans le système ABO », au sens de la présente invention, une jaunisse accomagnant la maladie hémolytique du nouveau-né (MHN) par incompatibilité ABO (encore appelée maladie hémolytique ABO, maladie hémolytique néonatale avec incompatibilité ABO ou jaunice hémolytique isoimmune due à une incompatibilité ABO). Cette jaunice est due à une accumulation de bilirubine dans plusieurs organes, la bilirubine étant le produit de dégradation de l'hème transporté dans le sang.

Plus particulièrement, les patients auxquels sont susceptibles d'être administrés la composition d'IgG décrite peuvent être des nouveaux-nés prématurés ou des nouveaux-nés nés à terme, par exemple depuis la naissance jusqu'au 28ème jour après la naissance. Ces bébés présentent généralement une hyperbilirubinémie due à la maladie hémolytique ABO, sont positifs à un test néonatal antiglobuline (test de Coombs indirect) et présentent une numération en réticulocytes élevée (supérieure ou égale à 10 %). Ces enfants peuvent être des garçons ou des filles.

La composition **utilisée pour** l'invention peut donc être également utilisée comme médicament pour le traitement de la maladie hémolytique du nouveau-né par incompatibilité ABO.

Les compositions d'IgG, ou concentrés d'IgG, tels que définis ci-dessus peuvent présenter une très faible teneur en IgG polyréactives, pouvant être par exemple comprise entre 0,01% et 0,1%, en particulier entre 0,07 et 0,1%. Dans ce cadre, la teneur d'IgG polyréactives signifie un pourcentage molaire ou massique. Cette teneur est déterminée par les méthodes décrites par la Demanderesse dans la demande de brevet EP 1 059 088.

Par exemple, la composition d'immunoglobulines G **utilisée pour** l'invention peut présenter, de manière avantageuse, des titres respectifs en anticorps anti-A et anti-B de 64 en inverse de dilution, ou compris entre 0 et 8, ou inférieurs à 4, par exemple inférieurs à 2, le test de Coombs étant mis en oeuvre avec une solution d'IgG de concentration intitiale ramenée à 30 g/l, et une teneur en IgG polyréactives, pouvant être par exemple comprise entre 0,01% et 0,1%, en particulier entre 0,07 et 0,1%.

On entend par « IgG, polyréactives », au sens de la présente invention, une fraction d'IgG contenue dans la composition d'IgG définie ci-avant, correspondant à la somme des anticorps naturels qui ne résultent pas d'une immunisation délibérée et qui expriment des affinités variables pour lés antigènes du soi, des anticorps anti-idiotypiques (c'est-à-dire dirigés contre la région variable d'autres anticorps), et des anticorps devenus polyréactifs à la suite des traitements reçus lors de différentes du procédé de purification de la composition d'IgG définie ci-avant. Avantageusement, la composition d'IgG utilisée dans l'invention se distingue des compositions des autres compositions d'IgG disponibles dans le commerce par leur quasi-absence de polyréactivité. Or, la Demanderesse a découvert de manière tout à fait surprenante que cette quasi-absence de polyréactivité des compositions d'IgG, combinée à des taux d'anticorps anti-A et anti-B très faibles de ces compositions, est une caractéristique importante pour le traitement de l'ictère néonatal par incompatibilité foetomaternelle dans le système ABO ou de la maladie hémolytique du nouveau-né par incompatibilité ABO. Ainsi, la composition d'IgG utilisée dans l'invention est efficace comme médicament pour le traitement de l'ictère néonatal par incompatibilité foetomaternelle dans le système ABO ou de la maladie hémolytique du nouveau-né par incompatibilité ABO, tout en évitant les réactions indésirables sur le plan des hématies (notamment par l'appauvrissement en anticorps anti-A et anti-B) et en réduisant les réactions secondaires indésirables qui résultent de la présence d'IgG polyréactives (notamment les fièvres, nausées, céphalées).

La composition **utilisée pour** l'invention peut en outre comprendre un ou plusieurs stabilisants.

On entend par « stabilisant », au sens de la présente invention, un composé permettant la conservation de la composition d'IgG dans le temps. Le stabilisant peut notamment permettre la conservation de la composition d'IgG pour une durée définie. Par ailleurs, le stabilisant est avantageusement compatible avec un usage thérapeutique.

Le stabilisant peut être avantageusement un de ceux mis au point par la Demanderesse dans sa demande de brevet WO 2004/091656, à savoir un mélange d'un sucre alcoolique, de préférence le mannitol, le sorbitol ou leurs isomères, de glycine et d'un détergent non ionique, tel que le Tween^{™}80, le Tween^{™}20, le Triton ® X100 ou le Pluronic ® F68, tous trois étant des composés acceptables sur le plan pharmaceutique.

Les concentrations finales en mannitol dans la composition d'IgG peuvent être comprises entre 30 g/l et 50 g/l ppm, et celles de la glycine, entre 7 g/l et 10 g/l. Les concentrations de ces composés représentent les concentrations finales dans les compositions d'IgG.

Avantageusement, les concentrations de la formulation ont été déterminées par la Demanderesse pour stabiliser les formes liquides et/ou lyophilisées.

La composition **utilisée pour** l'invention peut être administrée par voie intraveineuse, ou par voie sous-cutanée. A cet effet, les concentrés d'IgG selon l'invention doivent être viralement sécurisés, par exemple par un traitement classique au solvant-détergent connu de l'art antérieur, utilisant par exemple un mélange Tween® 80/TnBP ou Triton ® X 100 /TnBP, et/ou subir des étapes de filtration pour éventuellement éliminer les virus et/ou autres macromolécules qui n'auraient pas été éliminés par le traitement virucide de solvant-détergent, tels que le prion, agent responsable des encéphalopathies spongiformes transmissibles.

Les concentrés d'IgG **utilisés pour** l'invention peuvent également être soumis à une étape de nanofiltration.

La composition **utilisée pour** l'invention peut être formulée de manière à se présenter sous forme liquide ou lyophilisée en présence de stabilisants appropriés, et être stockée dans l'attente d'une utilisation ultérieure.

Avantageusement, la composition est injectable par voie intraveineuse.

Dans ce mode de réalisation, la composition **utilisée pour** l'invention peut être une solution pour injection, par exemple une solution d'Immunoglobuline humaine normale pour injection pour utilisation intraveineuse à 5g/100 ml (5%).

Avantageusement, la solution pour injection peut être dosée à 1g/100 ml (1 %), ou à 2g/100 ml (2 %), ou à 3g/100 ml (3 %), ou à 4 g/100ml (4 %), ou à 5 g/100 ml (5 %).

Avantageusement, les doses en IgG de la solution injectable peuvent être comprises entre 1g/100 ml et 5 g/100 ml, ou entre 2g/100 ml et 5g/100 ml, entre 3g/100ml et 5g/100 ml, ou égal à 5g /100 ml.

La composition permettant de réaliser l'invention peut être administrée de manière concomittante, ou consécutivement à un traitement par photothérapie, par exemple à une quantité comprise entre 500 mg/kg et 2000 mg/kg. Il est possible de commencer par une administration de 500 mg/kg, puis, si le taux de bilirubine ne diminue pas (ce qui peut être testé par un test connu de bilirubinémie), d'augmenter la dose par paliers de 500 mg/kg jusqu'à normalisation du taux de bilirubine. Cette administration peut être répétée.

La composition permettant de réaliser l'invention peut être administrée sans photothérapie parallèle. La quantité administrée peut être comprise entre 500 mg/kg et 2000 mg/kg. Il est possible de commencer par une administration de 500 mg/kg, puis, si le taux de bilirubine ne diminue pas (ce qui peut être testé par un test connu de bilirubinémie), d'augmenter la dose par paliers de 500 mg/kg jusqu'à normalisation du taux de bilirubine. Cette administration peut être répétée.

Une composition adaptée à la réalisation de l'invention peut être identique à celle qui est décrite dans le document WO 2007/077365.

La composition d'IgG peut être obtenue par tout procédé connu de l'homme du métier.

Notamment, la composition peut être obtenue par un procédé comprenant les étapes suivantes :
a) préparation d'une composition d'IgG par fractionnement éthanolique et/ou par séparation chromatographique, comprenant une étape d'inactivation virale,
b) chromatographie d'immunoaffinité par percolation de la composition d'IgG sur un mélange de supports dont les matrices sont greffées de groupes oligosaccharides antigéniquement similaires aux groupes sanguins A et B, et
c) filtration d'élimination de virus et/ou de particules de taille supérieure à 20 nm.

Avantageusement, un tel procédé est décrit dans le document WO 2007/077365.

Un tel procédé peut très avantageusement être mis en oeuvre à l'échelle industrielle. De plus, la combinaison d'étapes aboutissant à la préparation de compositions d'IgG et d'une étape spécifique d'élimination d'anticorps anti-A et anti-B permet d'obtenir une composition d'IgG, à usage thérapeutique, comprenant en outre, de préférence, un taux d'IgG polyréactives inférieur à 0,1% par rapport à la teneur totale en IgG. De surcroît, une telle composition comprend un titre en AcaA et AcaB indésirables bien inférieur à la valeur limite basse de l'essai décrit dans la Pharmacopée Européenne, c'est-à-dire inférieur à 64 (en dilution inverse) et même donnant un résultat négatif par la mise en oeuvre du test ICT avec de tels échantillons non dilués, c'est-à-dire un titre égal à 0.

De préférence, l'étape a) du procédé peut être en elle-même un procédé d'obtention de concentrés d'IgG, tels que ceux bien connus de l'homme du métier. Il s'agit d'un fractionnement éthanolique développé par Cohn et al. (Cohn et al. 1946, J. Am. Chem. Soc. 68, 459 ; Oncley et al. 1949, J. Am. Chem. Soc. 71, 541 [11]) ou bien d'une séparation chromatographique, telle que décrite par exemple dans EP 0 703 922 et WO 99/64462. On préfère tout particulièrement les procédés mis au point par la Demanderesse dans les demandes de brevet WO 94/29334 et WO 02/092632, et tout particulièrement celui décrit dans WO 02/092632. Dans ce cas, l'étape a) du procédé de l'invention comprend une prépurification par précipitation de contaminants lipidiques à partir d'un plasma sanguin ou d'une fraction de plasma sanguin enrichie en IgG, une chromatographie unique sur un support de résine échangeuse d'anions effectuée à pH alcalin, une élution sélective des IgG en une étape par un tampon approprié à pH compris entre 4 et 7.

On entend par « contaminants lipidiques », au sens de la présente invention, les constituants du plasma autres que les immunoglobulines.

On entend par « fraction de plasma sanguin enrichie en IgG », au sens de la présente invention, une fraction de plasma ayant déjà subi des étapes de purification, de manière à augmenter la concentration en IgG de cette fraction.

On entend par « chromatograhie unique », au sens de la présente invention, une étape de chromatographie non répétée ultérieurement.

On entend par « élution sélective des IgG en une étape », au sens de la présente invention, une étape d'élution permettant d'éluer en majeure partie les immunoglobulines G.

Aux fins de la présente invention, les tampons permettant l'élution sélective des IgG en une étape peuvent être tout tampon bien connu de l'homme du métier.

L'étape a) du procédé comprend un traitement d'inactivation de virale, de préférence effectué par solvant-détergent, comme décrit par Horowitz dans le brevet US 4 764 369. Il sera en particulier judicieusement mis en oeuvre, le cas échéant, avant l'étape chromatographique subséquente permettant d'éliminer notamment les résidus chimiques de ce traitement.

Ce concentré est ensuite soumis à une étape chromatographique d'immunoaffinité sur un mélange de deux supports greffés de groupes antigéniquement similaires aux groupes sanguins A et B, de préférence sur une colonne remplie d'un tel mélange de supports. De préférence, le support chromatographique est constitué d'une matrice en polymère naturel réticulé, de type agarose, sur laquelle sont greffés des espaceurs ou des bras de couplage, étant à leur tour greffés, avec des oligosaccharides représentant avantageusement des trisaccharides (oligosaccharides) correspondants aux épitopes des groupes sanguins A et B.

On entend par « groupes oligosaccharides antigéniquement similaires aux groupes sanguins A et B », au sens de la présente invention, des groupes oligosaccharides reconnus par les mêmes anticorps ou les mêmes immunoglobulines que les groupes sanguins A et B.

En particulier, de très bons résultats sont obtenus en mettant en oeuvre un tel support dont les trisaccharides, correspondant à l'épitope du groupe sanguin A, présentent la structure N-acétylgalactosamine (GaINAc) - Galactose (Gal) - Fucose (Fuc), et ceux correspondant à l'épitope du groupe sanguin B, présentent la structure Galactose-Galactose-Fucose. Un tel support représente très avantageusement un gel ou résine disponible dans le commerce sous la dénomination GLYCOSORB ABO ®, provenant de chez Glycorex Transplantation AS (Suède).

A titre d'exemple, si ce support est utilisé, le trisaccharide correspondant à l'épitope du groupe sanguin A présente la structure ci-dessous : 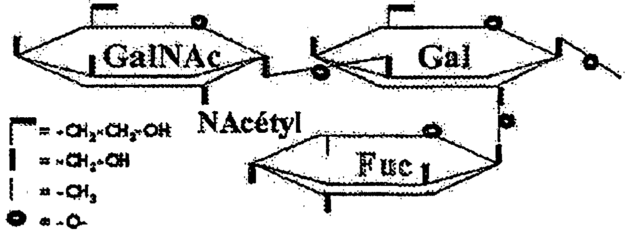
N-acétylgalactosaMine (GaINAc)
Galactose (Gal)
Fucose (Fuc).

A titre d'exemple, si ce support est utilisé, le trisaccharide correspondant à l'épitope du groupe sanguin B présente la structure ci-dessous :

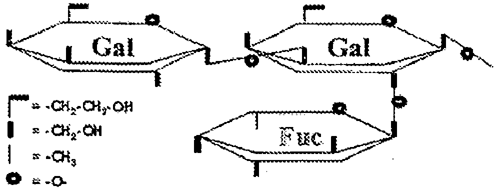

On entend par « support », au sens de la présente invention, un matériau inerte servant à supporter la matrice. Avantageusement, à un support est greffé une matrice, portant un type de groupes fonctionnels, à savoir un type de groupes oligosaccharides.

Ces supports sont bien connus de l'homme du métier.

La matrice peut être toute matrice adaptée. Ces matrices sont bien connues de l'homme du métier. On peut notamment donner comme exemple des matrices de sépharose, par exemple GLYCOSORB ABO (Glycorex Transplantation).

Il est possible d'utiliser la matrice GLYCOSORB ABO (Glycorex Transplantation), qui est greffée avec des trisaccharides des groupes sanguins A et B.

On entend par « mélange de supports », au sens de la présente invention, le mélange de supports dont certains portent la matrice greffée avec les oligosaccharides antigéniquemet similaires au groupe sanguin A, et dont d'autres portent la matrice greffée avec les oligosaccharides antigéniquemet similaires au groupe sanguin B.

Les différents types de matrices peuvent donc se trouver en proportions variables.

Avantageusement, le mélange de supports greffés de groupes antigéniguement similaires au groupe sanguin A et groupe sanguin B est en une proportion respective comprise entre 25/75 et 75/25 (v/v). Il est en effet possible d'ajuster la proportion des deux supports dans la colonne à la population des donneurs selon la répartition des groupes sanguins de celle-ci. Dans le cadre d'une utilisation habituelle, la colonne sera remplie de préférence avec un mélange de 50/50 (v/v) en chaque support spécifique ci-dessus. On peut utiliser des colonnes analytiques de 15 à 25 cm de longueur et de 0,5 à 1 cm de diamètre. Dans le cas d'une mise en oeuvre à l'échelle pilote, on peut utiliser des colonnes de 40 à 60 cm de longueur et de 40 à 60 mm de diamètre. Dans ce cas, il est possible de charger la colonne de 600 ml de support d'immunoaffinité.

Un tel support peut être stocké en NaOH 1 M entre deux cycles d'utilisation. Avant utilisation, il est lavé par de l'eau.

On peut ensuite charger la colonne chromatographique d'immunoaffinité en concentré d'IgG de préférence à raison de 0,2 à 4 litres, en particulier de 1 à 2 litres, par millilitre de support. La spécificité d'un tel support ne nécessite pas un conditionnement préalable de la fraction d'IgG, c'est-à-dire que toute fraction ou concentré d'IgG obtenue par les techniques de fractionnement de plasma de l'art antérieur peut convenir.

La percolation du concentré ne fait pas intervenir le mécanisme d'élution. Par conséquent, quelle que soit la manière dont est obtenu le concentré d'IgG, il peut être percolé à travers la colonne, éventuellement grâce a une pompe. Cette percolation permet la rétention des AcaA et AcaB et des IgG polyréactives. Avantageusement, la colonne est ensuite lavée par de l'eau pour récupérer les IgG encore présentes dans le volume mort de la colonne.

Après percolation du concentré d'IgG, on obtient une fraction d'IgG appauvrie en AcaA et AcaB, ainsi qu'en IgG polyréactives issues du procédé de fabrication. En effet, les AcaA et AcaB sont retenus sur leur motif antigénique du support chromatographique qui en modifie la conformation.

L'affinité de ces IgG polyréactives retenues de façon secondaire est beaucoup moins importante que celle des AcaA et AcaB. Il est possible de les éluer de manière fractionnée, après le passage des IgG, par l'utilisation d'un tampon d'élution contenant, par exemple, un sel de métal alcalino-terreux de concentration comprise entre 0,1 et 1, 5 M, à un pH de 3-8,6.

La colonne chromatographique et le support peuvent être ensuite lavés avec une solution acide, telle que de glycine-HCl, pH 2,8, pour la désorption des AcaA et AcaB retenus sur le support. Ce support est ensuite rincé avec de l'eau et traité avec une solution de NaOH 1 M.

Le concentré d'IgG très appauvri en AcaA, AcaB est ensuite soumis à une filtration d'élimination de virus résistants au traitement par solvant-détergent et/ou autres particules de taille supérieure à 20 nm, tels que les prions, les polymères d'IgG engendrés lors d'étapes de sa fabrication, les lipopolysaccharides en micelles, les acides nucléiques et les protéines agrégées. Un tel traitement représente avantageusement la nanofiltration, mise en oeuvre par des filtres de porosité décroisssante de 100 à 15 nm, en particulier sur trois filtres disposés en série et de seuils de rétention décroissants, de 100, 50 et 20 nm.

Avantageusement, le procédé comprend une étape d'inactivation virale.

On entend par « inactivation virale », au sens de la présente invention, toute méthode ou étape permettant d'inactiver, c'est-à-dire de dénaturer de manière efficace les particules virales tout en respectant la fonctionnalité des protéines plasmatiques.

Les méthodes d'inactivation virale sont bien connues de l'homme du métier. Parmi ces méthodes, une étape d'inactivation virale peut être choisie parmi : l'étape de solvant-détergent, pasteurisation.

Avantageusement, l'étape d'inactivation virale peut être effectuée par une étape de solvant-détergent.

La fraction d'IgG ainsi récoltée est déjà suffisamment concentrée, et peut ensuite subir des étapes de concentration supplémentaire par ultrafiltration et de filtration stérilisante.

Le procédé peut comprendre, après l'étape b), des étapes de concentration par ultrafiltration et de filtration stérilisante.

Avantageusement, l'étape de filtration stérilisante peut être effectuée par nanofiltration.

Le procédé peut comprendre, après l'étape c), une étape supplémentaire d'ajout de stabilisants afin, d'une part, d'assurer la stabilité des concentrés d'IgG en cours de conservation dans le temps et, d'autre part, permettre une lyophilisation évitant la dénaturation des IgG dans les diverses phases associées à celle-ci. De préférence, il sera ajouté une formulation stabilisante unique, pharmaceutiquement acceptable, répondant à l'objectif d'assurer la stabilisation des deux formes de conservation envisagées des IgG à la fois, à savoir sous forme liquide ou lyophilisée, et de conserver, voire améliorer, l'efficacité thérapeutique de ces IgG, comme décrit dans la demande de brevet WO 2004/091656.

Les compositions d'IgG sont éventuellement soumises à une étape ultérieure de concentration par ultrafiltration, puis à une filtration stérilisante et peuvent être conditionnées dans des flacons et conservées de préférence à des températures voisines de 4°C.

Une méthode de dosage peut être utilisée pour doser les anticorps anti-A et anti-B de la composition d'IgG décrite dans l'invention.

Une telle méthode de dosage des anticorps anti-A et/ou anti-B dans les concentrés d'IgG peut comprendre les étapes consistant à :
a) préparer et calibrer une suspension d'hématies du groupe sanguin O Rhésus +,
b) préparer des solutions d'anticorps anti-D monoclonal dans une plage de concentrations allant de 0 à 200 ng/ml dans un tampon biologiquement acceptable,
c) mettre en contact lesdites hématies avec les solutions d'anticorps anti-D monoclonal, et incuber les mélanges d'hématies ainsi obtenus pendant une durée prédéterminée,
d) ajouter dans chaque mélange d'hématies un fragment d'anticorps anti-IgG humaines F(ab')2 marqué au moyen d'un fluorochrome et incuber lesdites hématies,
e) soumettre chaque mélange d'hématies obtenu à l'étape d) à une cytométrie de flux,
f) élaborer une courbe étalon de la concentration en anticorps monoclonal anti-D en fonction de la fluorescence.

Il est ensuite possible de doser les anticorps anti-A et anti-B en mettant en oeuvre la méthode suivante :
a) préparer et calibrer une suspension d'hématies du groupe sanguin A, B-,
b) préparer des solutions d'anticorps anti-D monoclonal dans une plage de concentrations allant de 0 à 200 ng/ml dans un tampon biologiquement acceptable,
c) mettre en contact lesdites hématies avec des échantillons de solutions d'IgG, et incuber les mélanges d'hématies ainsi obtenus pendant une durée prédéterminée,
d) ajouter dans chaque mélange d'hématies un fragment d'anticorps anti-IgG humaines F(ab')2 marqué au moyen d'un fluorochrome et incuber lesdites hématies,
e) soumettre chaque mélange d'hématies obtenu à l'étape d) à une cytométrie de flux,
g) déterminer le titre en anticorps anti-A et/ou anti-B dans les concentrés d'IgG au moyen de la courbe étalon établie pour doser l'anti-D, avantageusement exprimée en nanogramme/millilitre.

Un mode de mise en oeuvre d'une telle méthode de détermination du titre en anticorps anti-A et/ou anti-B peut comprendre la préparation d'une suspension d'hématies à 1 % (v/v) du groupe sanguin A, B et/ou O dans un tampon PBS, de pH compris entre 7,0 et 7,4, contenant de 0,8 à 1,5 % en poids de sérum-albumine bovine BSA. Les hématies de la suspension sont comptées dans un dispositif de cytométrie de flux habituel, dont la mise en oeuvre est connue de l'homme du métier, puis de façon à calibrer la suspension à 37 à 43.106 hématies/ml de suspension. On prépare des solutions d'anticorps anti-D monoclonal, dont les concentrations sont comprises dans la plage allant de 0 à 200 ng/ml de tampon, de préférence un tampon PBS, de pH compris entre 7,0 et 7,4, contenant le cas échéant de 0,8 à 1,5 % en poids de sérum-albumine bovine BSA. Chaque solution ainsi préparée est dosée par absorptiométrie afin de déterminer son coefficient d'extinction molaire ( 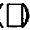 ).

Les compositions d'IgG sont ensuite ajustées à une concentration comprise dans la gamme de valeurs allant de 1 à 5 mg/ml, de préférence à 1 mg/ml, au moyen d'un tampon PBS, de pH compris entre 7,0 et 7,4, contenant de 0,8 à 1,5 % en poids de sérum-albumine bovine BSA.

On place un volume de 50 à 100 µl de la suspension d'hématies de chaque groupe sanguin dans chaque puit d'une microplaque, par exemple de 96 puits, puis de 50 à 100 µl de solutions d'IgG dans cette suspension d'hématies, ou de 50 à 100 µl de solutions d'anticorps anti-D dans cette suspension d'hématies. L'ensemble est mis à incuber pendant des durées comprises entre 1h30 et 2h30, en particulier 2h, à des températures habituellement comprises entre 30 et 40°C, de préférence 37°C.

Les différents mélanges d'hématies ainsi obtenus sont ensuite de préférence lavés avec le tampon PBS contenant de la BSA précédente et est centrifugées, puis on ajoute dans chaque mélange d'hématies, contenus dans un puit de microplaques, de 50 à 100 µl d'anticorps F(ab')2 de chèvre anti-IgG humaines marquées d'un fluorochrome, tel que par exemple la phycoérythrine, présents dans du tampon PBS et de BSA défini précédemment.

L'incubation de l'ensemble est mise en oeuvre pendant environ 20-30 minutes à l'abri de la lumière.

Les différents mélanges d'hématies ainsi obtenus sont ensuite lavés et soumis à une cytométrie de flux mise en oeuvre avec tout appareil approprié disponible dans le commerce comportant un dispositif de détection de fluorescence des composés analysés.

L'intensité moyenne de fluorescence (IMF) est rapportée en fonction de la concentration en anticorps monoclonal anti-D et l'équation de la droite de régression est obtenue au moyen du logiciel Excel. Puis pour chaque échantillon, la concentration en équivalent anticorps anti-D est obtenue en utilisant l'équation linéaire de la droite de régression. Les échantillons ayant été dosés en triple, la moyenne de la concentration est établie et le coefficient de variation est calculé par le logiciel Excel.

On en déduit la teneur en anticorps anti-A et anti-B dans les concentrés d'IgG **utilisés pour** l'invention qui est celle avantageusement donnée précédemment.

De préférence, une méthode de dosage des AcaA et AcaB des concentrés d'IgG ci-dessus est effectuée par cytométrie de flux adaptée au contexte de l'invention, dont le principe repose sur l'utilisation d'hématies humaines de groupe A ou B, selon la détermination spécifique du titre des AcaA et AcaB voulue, mettant en oeuvre la détection d'un signal de fluorescence proportionnel à la teneur en ces anticorps.

Une telle méthode de dosage comprend les étapes consistant à :
a) préparer et calibrer une suspension d'hématies du groupe sanguin A ou B,
b) mettre en contact lesdites hématies avec des échantillons dilués de solutions d'IgG, et incuber le mélange ainsi obtenu pendant une durée prédéterminée,
c) incuber lesdites hématies en présence d'un anticorps anti-IgG marqué au moyen d'un fluorochrome, et
d) soumettre la suspension d'hématies obtenue à l'étape c) à une cytométrie de flux.

On prépare une suspension d'hématies à 1% (v/v) du groupe sanguin A ou B dans un tampon PBS, de pH compris entre 7,0 et 7,4, contenant de 0,8 à 1,5 % en poids de sérum-albumine bovine BSA. Les hématies de la suspension sont comptées dans un dispositif de cytométrie de flux habituel, dont la mise en oeuvre est connue de l'homme du métier, puis de façon à calibrer la suspension à 37 à 43.10 hématies/ml de suspension.

On place un volume de 50 à 100 µl de la suspension dans chaque puit d'une microplaque de 96 puits, puis de 50 à 100 µl de différentes solutions d' IgG diluées de deux en deux à partir d'une solution de 30 g/l jusqu'à obtenir une solution d'IgG à 0,234 g/l.

L'ensemble est mis à incuber pendant des durées comprises entre 1h30 et 2h30, en particulier 2h, à des températures habituellement comprises entre 30 et 40°C, de préférence 37°C.

Les hématies sont ensuite lavées avec le tampon PBS contenant de la BSA précédente et est centrifugées puis on ajoute dans chaque puit de 50 à 100 µl d'anticorps F(ab')2 de chèvre anti-IgG humaines marquées d'un fluorochrome, tel que par exemple la phycoérythrine.

L'incubation de l'ensemble (l'étape c)) est mise en oeuvre pendant environ 20-30 min à l'abri de la lumière.

La suspension ainsi obtenue est ensuite lavée et soumise à une cytométrie de flux mise en oeuvre avec tout appareil approprié disponible dans le commerce comportant un dispositif de détection de fluorescence des composés analysés.

A titre d'exemple, les teneurs en anticorps anti-A et B de trois concentrés d'IgG dénommés B1, B2 et B3 , préparés respectivement par fractionnement éthanolique selon la méthode de Cohn (B1), selon la demande de brevet WO 02/092632 (B2) et selon la demande de brevet WO 02/092632 suivie d'une chromatographie d'immunoaffinité (B3) pour l'appauvrissement en anticorps anti-A et anti-B, sont présentées dans le Tableau 1 qui suit. Les résultats sont présentés par rapport au titre témoin en anticorps anti-A et anti-B de l'échantillon B1 dont la teneur en ces anticorps a été fixée arbitrairement à 1, à titre de référence.

**Tableau 1.**

| Echantillons | Teneur en anticorps anti-A | Teneur en anticorps anti-B |
|---|---|---|
| B1 | 1 | 1 |
| B2 | 3,65 | 3,85 |
| B3 | 0,68 | 0,52 |

Les résultats de ce tableau montrent tout d'abord que les teneurs en anticorps anti-A et anti-B des concentrés d'IgG (BI), préparés selon la méthode de Cohn, en contiennent environ quatre fois moins que les concentrés d'IgG (B2), préparés selon la méthode décrite dans WO 02/092632. De plus, le traitement ultérieur de ces concentrés d'IgG par des colonnes d'immunoaffinité spécifiques réduisent le titre en anticorps anti-A d'un facteur voisin de 5 et d'un facteur voisin de 7 en anticorps anti-B (B3).

Une autre méthode de détermination de la teneur en anticorps anti-A et anti-B pouvant être avantageusement mise en oeuvre consiste en la lyse par le complément in vitro, connue de l'homme du métier, mais qui a été spécifiquement mise au point pour les besoins de l'invention. Une telle méthode de dosage comprend les étapes consistant à :
a) procéder à un radiomarquage d'une suspension d'hématies papaïnées choisies parmi les groupes sanguins A, B, AB et O, préalablement comptées, par un marqueur radioactif approprié,
b) mettre en contact les hématies radiomarquées avec des échantillons de concentrés d'IgG en un volume prédéterminé,
c) ajouter un volume identique à celui de l'étape b) de sérum normal du groupe sanguin AB,
d) incuber le mélange obtenu à l'étape c) pendant une durée prédéterminée, et
e) mesurer la radioactivité de la solution incubée ainsi obtenue.

On prépare une suspension d'hématies papainées à 1% (v/v) du groupe sanguin A, B, AB ou O qui est ensuite comptée dans une cellule de Malassez pour obtenir 106 hématies. On ajoute 100 µCi de 51 Cr (1 volume pour 1 volume d'hématies). On incube l'ensemble entre 1 et 2 heures, et les hématies radiomarquées sont ensuite lavées entre 4 et 6 fois.

Les hématies radiomarquées sont ensuite mises en contact avec des échantillons de concentrés d'IgG, à une concentration comprise de préférence entre 1 et 3 mg/ml, en particulier 1,2 mg/ml pour 4-6.106 hématies radiomarquées, dans un volume par exemple de 100 µl.

Un volume identique au précédent, par exemple de 100 µl, de sérum normal de groupe sanguin AB est ensuite ajouté au mélange précédent pour apporter les différents facteurs de la voie de complément.

Le mélange réactionnel ainsi obtenu est ensuite incubé, de préférence pendant des durées comprises entre 3 et 5h, en particulier 4h, à des températures habituellement comprises entre 30 et 40°C, de préférence 37°C.

Le mélange réactionnel est ensuite, de préférence, centrifugé, et on procède à une mesure de la radioactivité de la solution incubée selon des dispositifs appropriés disponibles dans le commerce. La radioactivité mesurée de la solution est proportionnelle au taux d'hémolyse des hématies traitées et, par conséquent, à la teneur en anticorps anti-A et anti-B.

A titre d'exemple, les taux d'hémolyse des hématies des groupes sanguins A, B et AB obtenus en considérant un concentré d'IgG de l'invention (B3) et un concentré d'IgG de l'art antérieur (C1) ayant des taux d'hémolyse les plus faibles parmi les concentrés disponibles dans le commerce, sont indiqués au Tableau 2 qui suit.

**Tableau 2**

| Taux d'hémolyse d'hématies (%) | B3 | C1 (art antérieur) |
|---|---|---|
| Groupe A | 6 | 13 |
| Groupe B | 5 | 11 |
| Groupe AB | 6 | 13 |

Un autre objet **décrit** est utilisation d'une composition telle que définie ci-dessus pour la fabrication d'un médicament destiné au traitement de l'ictère nénonatal par incompatibilité foetomaternelle dans le système ABO.

Tous les aspects de l'invention ci-dessus mentionnés sont adaptés à cette utilisation et font partie de cet autre objet de l'invention.

Un autre objet **décrit** est une méthode de traitement de l'ictère nénonatal par incompatibilité foetomaternelle dans le système ABO ou de la maladie hémolytique néonatale ABO, comprenant l'administration d'une composition d'IgG telle que précédemment définie.

Dans ce mode de réalisation, la composition **utilisée pour** l'invention peut être une solution pour injection, par exemple une solution d'Immunoglobuline humaine normale pour injection pour utilisation intraveineuse à 5g/100 ml (5%).

Avantageusement, la solution pour injection peut être dosée à 1g/100 ml (1 %), ou à 2g/100 ml (2 %), ou à 3g/100 ml (3 %), ou à 4 g/100ml (4 %), et avantageusement jusqu'à 5 g / 100ml (5 %).

Avantageusement, les doses en IgG de la solution injectable peuvent être comprises entre 1g/100 ml et 5 g/100 ml, ou entre 2g/100 ml et 5g/100 ml, ou entre 3g/100ml et 5g/100 ml.

La composition permettant de réaliser l'invention peut être administrée de manière concomittante, ou consécutivement à un traitement par photothérapie, par exemple à une quantité comprise entre 500 mg/kg et 2000 mg/kg. Cette administration peut être répétée.

Tous les aspects de l'invention décrits précédemment sont adaptés à cette méthode de traitement.

D'autres avantages pourront encore apparaître à l'homme du métier à la lecture des exemples ci-dessous.

### EXEMPLES

### Exemple 1: préparation de compositions d'IgG appauvries en anticorps anti-A et anti-B

Un échantillon de concentré d'IgG à 40 g/l (B2) est obtenu par la mise en oeuvre du procédé décrit dans WO 02/092632.

Une colonne chromatographique de 50 cm de longueur et de 44 mm de diamètre est remplie d'un mélange 50/50 (v/v) de support GLYCOSORB ABO greffé par des trisaccharides correspondants aux épitopes du groupe sanguin A et du groupe sanguin B, puis est soumise à une étape préalable de lavage par 1200 ml d'eau.

On injecte du concentré d'IgG B2 à raison de 0,2 l/ml de support par l'intermédiaire d'une pompe. Une fois que ce volume a été percolé à travers la colonne, celle-ci est lavée par un volume minimal d'eau pour préparation injectable (PPI) afin de récupérer les IgG présentes dans le volume mort de la colonne.

On récupère un concentré d'IgG B3 a environ 40 g/l appauvri en AcaA, AcaB et en IgG polyréactives qui est soumis à une ultrafiltration de façon que le concentré soit à 60 g/l et à une nanofiltration d'élimination de virus sur trois filtres disposés en série et de seuils de rétention décroissants de 100, 50 et 20 nm.

La dissolution des excipients stabilisants constitués d'un mélange de glycine à 7 g/l, de mannitol à 30 g/l et de 20 ppm de Tween□ 80 dans le concentré d'IgG à 60 g/l est suivie d'un ajustement de la concentration en IgG à 50 g/l au moyen d'eau PPI, puis le concentré est filtré stérilement et réparti en flacons.

### Exemple 2 : quantification des anti-A/B dans les IGIV

### 1) Principe du dosage

### 1.1) Préparation des hématies humaines

Les suspensions d'hématies humaines de groupe A rhésus +, B rhésus + ou O rhésus + sont normalisées à la concentration de 40 x 106 hématies/ml dans du tampon PBS + 1 % BSA à pH = 7,4.

### 1.2) Préparation de la gamme d'anti-D monoclonal

Une préparation d'anti-D monoclonal (appelés R297) est dosée en Densité Optique (DO) à 280nm contre son tampon de PBS, de pH 7,4. Le coefficient d'extinction molaire ( 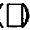 ) de la protéine est calculé par rapport à sa composition en différents acides aminés et la concentration (C) en anti-D monoclonal est obtenue en appliquant la formule C = DO/□ avec 1 = largeur de la cuve pour effectuer la mesure de la DO.

Une gamme de 0 à 200 ng/ml d'anticorps monoclonal anti-D est réalisée en 12 points (200 ; 150 ; 100 ; 75 ; 50 ; 25 ; 12,5 ; 6,25 ; 3,13 ; 1,56 ; 0,78 et 0 ng/ml).

### 1.3) Préparation des solutions d'immunoglobulines

Différentes immunoglobulines intraveineuses, disponibles dans le commerce, ont été étudiées. Les caractéristiques principales de ces immunoglobulines sont détaillées dans le tableau ci-dessous :

| Désignation | Fournisseur | Concentration |
|---|---|---|
| Immunoglobuline polyvalente | A | 50 g/l |
| Immunoglobuline polyvalente | B | 50 g/l |
| Immunoglobuline humaine itraveineuse à 10% | C | 100 g/l |
| IgNG 2 (*) | LFB | 50 g/l |
| IgNG 1 (**) | LFB | 50 g/l |
| TEGELINE□ | LFB | 50 g/l |

| | | |
|---|---|---|
| * réalisées au moyen de la mise en oeuvre du procédé décrit dans WO 02/092632 suivi de l'étape d'immunoaffinité décrite dans l'exemple 1 ** réalisées au moyen de la mise en oeuvre du procédé décrit dans WO 02/092632, sans étape d'immunoaffinité telle que décrite dans l'exemple 1 | | |

Les différentes préparations d'immunoglobulines sont ajustées à la concentration de 1 mg/ml au moyen d'un tampon PBS + 1 % BSA à pH=7,4.

### 1.4) Sensibilisation des hématies

Dans un micro plaque à fond rond, sont déposées dans les puits :
- 50 µl de la suspension d'hématies A rhésus +, B rhésus + ou O rhésus + à 40 x 106 hématies/ml,
- 50 µl de la gamme anti-D ou 50 µl des échantillons (IgIV) à doser.
Les échantillons à doser sont déposés en triple.

Les plaques sont ensuite incubées 2 heures à 37°C sous agitation.

### 1.5) lavages

Les plaques sont centrifugées 1 minute à 770 g. Le surnageant est écarté par retournement et puis 200 µl de PBS + 1% BSA est ajouté dans chaque puits. L'opération est renouvelée 3 fois.

### 1.6) Ajout du conjugué et lavages

Un F(ab')2 de chèvre anti-IgG humaines (Fc spécifique) marqué à la phycoérythrine (PE) (Beckman Coulter Ref : PN IM0550) est dilué au l/20ème dans le tampon PBS + 1 % BSA pH=7,4 puis 50 µl de la solution sont déposés dans chaque puits. La plaque est ensuite incubée 20 à 30 minutes à température ambiante à l'abri de la lumière. 3 lavages successifs sont réalisés tels que décrit au paragraphe 1- 5).

### 1.7) Lecture au cytomètre de flux

Les suspensions d'hématies sont lues au cytomètre de flux (Beckman Coulter FC500) selon un programme approprié.

La lecture est effectuée sur 50 000 événements et l'appareil calcul automatiquement l'intensité moyenne de fluorescence (IMF) de chaque point de gamme ou échantillon.

### 1.8) Interprétation des résultats

L' IMF est rapportée en fonction de la concentration en anticorps monoclonal anti-D et l'équation de la droite de régression est obtenue au moyen du logiciel Excel. Puis pour chaque échantillon, la concentration en équivalent anticorps anti-D est obtenue en utilisant l'équation linéaire de la droite de régression. Les échantillons ayant été dosés en triple, la moyenne de la concentration est établie et le coefficient de variation est calculé par le logiciel Excel.

### 2) Résultats :

### 2.1) Concentration en anticorps anti -A

| Nom | Hématies O rhésus + | Hématies A rhésus + (ng Ig anti-A/mg d'Ig) | CV% |
|---|---|---|---|
| Immunoglobuline polyvalente, A | ns | 55,4 | 4,5 |
| Immunoglobuline polyvalente, B | ns | 44,4 | 3,4 |
| Immunoglobuline humaine intraveineuse à 10 %, C | ns | 117,9 | 14,6 |
| IgNG 2 | ns | 22,2 | 5,5 |
| IgNG 1 | ns | 119,8 | 4,9 |
| TEGELINE | ns | 35,6 | 5,0 |

| | | | |
|---|---|---|---|
| ns = non significatif | | | |

### 2.2) Concentration en anticorps anti-B

| Nom | Hématies O rhésus + | Hématies B rhésus + (ng Ig anti-B/mg d'Ig) | CV (%) |
|---|---|---|---|
| Immunoglobuline polyvalente, A | ns | 64,0 | 0,9 |
| Immunoglobuline polyvalente, B | ns | 42,4 | 5,1 |
| Immunoglobuline humaine intraveineuse à 10%, C | ns | 89,0 | 20,5 |
| IgNG 2 | ns | 16 | 9,9 |
| IgNG 1 | ns | 155,2 | 4,8 |
| TEGELINE | ns | 44,2 | 8,0 |

| | | | |
|---|---|---|---|
| ns = non significatif | | | |

### 3) Conclusions :

L'étape d'affinité est réellement contributive pour l'élimination des anticorps anti-A et anti-B. Parmi différentes immunoglobulines étudiées présentes sur le marché, le produit IgNG2 est le produit contenant le moins d'anticorps anti-A et d'anticorps anti-B.

### Exemple 3 : Quantification des anti-A et anti-B du concentre d'IgG B obtenu à l'exemple 1

On prépare une suspension d'hématies à 1% (v/v) du groupe sanguin A dans un tampon PBS, de pH 7,4, contenant 1% en poids de serum-albumine bovine BSA. On prélève 50 µl de la suspension d'hématies et on les introduit dans un tube pour un cytomètre de flux Beckmann-Coulter Epies XL, ainsi que 50 µl d'une solution de marqueur interne mesurant le flux. La suspension est calibrée à 40.10⁶ hématies/ml.

Huit lots de solutions d'IgG sont préparés par dilution successive d'un facteur 2 du concentré d'IgG (v/v) (B3) obtenu à l'exemple 1, le lot le plus concentré étant à 30 g/l, le plus dilué à 0,234 g/l. On place ensuite un volume de 50 µl de la suspension dans chaque puit d'une microplaque de 96 puits, puis 50 µl des différentes solutions d'IgG diluées. L'ensemble est mis à incuber pendant 2h à une température de 37°C, sous agitation.

Chaque puit est ensuite lavé avec 200 µl de tampon PBS contenant de la BSA précédente et la microplaque est centrifugée pendant 1 minute à 2000 tours/min. Après élimination du surnageant, on ajoute dans chaque puit 50 µl d'une solution diluée à 1/20 par du PBS-BSA d'anticorps F(ab')2 de chèvre anti-IgG humaines marquées de fluorochrome de phycoérythrine (Beckmann Coulter).

L'incubation de l'ensemble est mise en oeuvre pendant 30 min à l'abri de la lumière.

La suspension ainsi obtenue est ensuite lavée comme précédemment.

Le culot de chaque puit est repris par 100 µl de PBS-BSA. Le volume contenu dans chaque puit de la microplaque est transféré dans un tube dans lequel sont ensuite ajoutés 500 µl de liquide de gaine Isoflow (Coulter) et est soumis à une cytométrie de flux mise en oeuvre avec le dispositif Coulter-Beckmann Epies XL comprenant des logiciels d'acquisition des données et d'exploitation des résultats. La fluorométrie est mesurée pour chaque échantillon.

On procède de la même manière avec des hématies de groupe sanguin B.

Ce mode opératoire est mis en oeuvre avec trois lots différents d'IgG (B3) et est également appliqué à trois lots différents d'IgG préparés par fractionnement éthanolique selon la méthode de Cohn (citée plus haut) (BI).

Les résultats obtenus figurent dans le Tableau 3 qui suit.

**Tableau 3**

| Echantillons | Teneur en anticorps anti-A | Teneur en anticorps anti-B |
|---|---|---|
| B1 | 1 | 1 |
| B2 (3 lots) | 3,80 ; 3,55 ; 3,59 | 3,80 ; 4,45 ; 3,31 |
| B3 (3 lots) | 0,66 ; 0,68 ; 0,69 | 0,33 ; 0,73 ; 0,50 |

### Exemple 4 : mesure de l'hémolyse des hématies

On prépare une suspension d'hématies papainées à 1% (v/v) du groupe sanguin A, B, AB ou O qui est ensuite comptée dans une cellule de Malassez pour obtenir 10 hématies. On ajoute 100 µCi de 51 Cr (1 volume pour 1 volume d'hématies). On incube l'ensemble pendant 1 heure et les hématies radiomarquées sont ensuite lavées 5 fois.

Les hématies radiomarquées sont ensuite mises en contact avec des échantillons de concentrés d'IgG (B2) obtenus à l'exemple 1, à une concentration de 1,2 mg/ml pour 5.106 hématies radiomarquées, dans un volume de 100 µl.

Un volume identique au précédent de 100 µl de sérum normal de groupe sanguin AB est ensuite ajouté au mélange précédent pour apporter les différents facteurs du complément.

Le mélange réactionnel ainsi obtenu est ensuite incubé, 4h, à une température de 37°C.

Le mélange réactionnel est ensuite centrifugé pendant 1 minute à 2000 tours/min, et on procède à une mesure de la radioactivité de la solution surnageante incubée, selon des dispositifs appropriés disponibles dans le commerce. La radioactivité mesurée de la solution est proportionnelle au taux d'hémolyse des hématies traitées et, par conséquent, à la teneur en anticorps anti-A et anti-B. On procède de façon identique avec des hématies des groupes sanguins B, AB et 0, étant tous de rhésus +, et avec un échantillon de sérum du groupe O+. Ce mode opératoire est mis en oeuvre avec trois lots différents d'IgG (B2). En outre, on applique le procédé à trois lots d'échantillons du commerce de concentrés d'IgG, notés C2 à C4, et un échantillon C5 de sérum du groupe O+, inclus comme témoin négatif.

La radioactivité mesurée de la solution est proportionnelle au taux d'hémolyse des hématies traitées et, par conséquent, à la quantité en anticorps anti-A et anti-B fixés sur les hématies.

Les résultats d'hémolyse obtenus sont présentés dans le Tableau 4 suivant.

**Tableau 4**

| Taux d'hémolyse d'hématies (%) | B3 | C2 | C3 | C4 | C5 |
|---|---|---|---|---|---|
| Groupe A+ | 6 | 16 | 13 | 30 | 34 |
| | 6,2 | 15,5 | 13,5 | 31 | 34,4 |
| | 6,5 | 16,3 | 13,2 | 31 | 34,6 |
| Groupe B+ | 5 | 13,1 | 11,2 | 25 | 34 |
| | 4,8 | 13,3 | 10,8 | 25,3 | 34,4 |
| | 5,4 | 13,4 | 10,9 | 25,4 | 34,6 |
| Groupe AB+ | 6 | 16 | 15,1 | 31 | 34 |
| | 5,9 | 15,7 | 15,1 | 31,4 | 34,4 |
| | 6,5 | 16,3 | 15,4 | 30,9 | 34,6 |
| Groupe O+ | 0,1 | 0,2 | 0,22 | 0,33 | 2 |
| | 0,15 | 0,25 | 0,24 | 0,32 | 2,2 |
| | 0,12 | 0,21 | 0,24 | 0,30 | 2,7 |

Les résultats obtenus montrent que le concentré d'IgG B3 qui a été soumis à une chromatographie d'affinité selon l'invention contient la plus faible quantité en AcaA et AcaB étant donné que le taux d'hémolyse des hématies provenant des différents groupes sanguins est le plus faible. On n'observe pas d'hémolyse avec des hématies de phénotype O+, inclus comme témoin négatif.

### Exemple 5 : mesure de la polyréactivité des concentrés d'IgG B2 (avant la chromatographie d'immunoaffinité) et après cette chromatographie (concentré d'IgG B3) décrite à l'exemple 1

La mesure de la polyréactivité de ces concentrés d'IgG est effectuée selon le brevet EP 1 059 088 en utilisant deux antigènes qui réagissent avec les IgG polyréactives. Il s'agit de la myosine et de l'albumine modifiée par des groupes dinitrophényle (Albumine DNP).

Le Tableau 5 présente les facteurs d'enrichissement des IgG polyréactives des échantillons B2, B3 et C4 de l'exemple 3 dont la teneur en IgG a été fixée arbitrairement à 1, à titre de référence.

Ces mesures ont été effectuées sur trois lots différents de-concentrés d'IgG considérés.

**Tableau 5**

| Echantillon | Myosine | Albumine DNP |
|---|---|---|
| B1 | 1 | 1 |
| B2 (3 lots) | 1,2 ; 0,8 ; 1,2 | 3,2 ; 1,0; 2,0 |
| B3 (3 lots) | 0,4 ; 0,4 ; 0,4 | 1,0 ; 1,0 ; 1,0 |
| C4 (3 lots) | 2,0 ; 2,0 ; 2,0 | 8,0 ; 6,0 ; 7,0 |

Les résultats indiquent que le concentré d'IgG B3 de l'invention contient de 5 à 8 fois moins d'IgG polyréactifs que le concentré de l'art antérieur C4.

### Exemple 6 : exemple comparatif sur l'efficacité des concentrés IgG (B3) appauvries en AcaA, AcaB, et en IgG polyréactives, avec des concentrés d'IgG (B1)

L'étude a porté sur des souris déficientes en récepteurs Fc 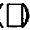 RI et Fc 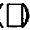 RIII traitées dans la perspective d'évaluation de l'activité immunomodulatrice des concentrés d'IgG selon l'invention. Ces animaux servent de modèle à un purpura thrombocytopénique. On utilise comme témoin un concentré d'IgG (B1) obtenu par fractionnement éthanolique selon Cohn.

On applique le protocole expérimental décrit par Teeling J. L et al (Blood, 15/08/2001, vol. 98, number 4, pp.1095-1099 [10]). Les plaquettes, effondrées par injection d'IgG monoclonales antiplaquettes de 9.10⁸/ml au niveau de 2.10⁸/ml, remontent à 7.10⁸/ml, chez les animaux traités par les concentrés d'IgG B1 et B3 à une dose thérapeutique de 1 g/kg.

L'activité immunomodulatrice du concentré d'IgG B3 selon l'invention n'a pas été modifiée par la mise en oeuvre de la chromatographie d'immunoaffinité.

### Exemple 7: Exemple de mise en oeuvre

### Evaluation de la dose à administrer et de la tolérance au traitement

La composition d'IgG permettant de réaliser l'invention, par exemple la composition B3 ou une autre composition d'IgG disponible sur le marché, est administrée à des nouveau-nés, garçons et filles, âgés de moins de 28 jours, atteints de la maladie hémolytique ABO, dont les symptômes sont une hyperbilirubinémie, un test d'antiglobulines néonatal direct positif (test de Coombs direct) et une numération en réticulocytes élevée (supérieure ou égale à 10 %).

Les critères d'exclusion des patients sont une déficience en IgA, la présence d'anticorps anti-IgE/IgG, l'indication d'une transfusion d'échange à la naissance avec un niveau de bilirubine dans le cordon ombilical supérieur ou égal à 4 mg, un anasarque foeto-placentaire (hydrops fetalis), une insuffisance cardiaque, et un traitement prénatal (IVIG maternelle et/ou transfusion in utero).

Le protocole de traitement est une administration d'IgG à 500 mg/kg et 2000 mg/kg (n= environ 10), seule ou en combinaisons avec une photothérapie, susceptible d'être réitérée.

Les critères dévaluation primaires sont la nécessité d'une transfusion d'échange, et le niveau de bilirubine total du sérum est mesuré 3 jours après la première administration de la composition.

Les principaux critères d'évaluation secondaires sont la durée totale de la photothérapie, la durée du temps d'hospitalisation, le développement d'une anémie tardive au 27ème jour post partum, et le niveau de bilirubine total du sérum 24 heures après la première administration de la composition d'immunoglobulines.

L'analyse comparative des résultats est réalisée au moyen de la méthode de Kaplan Meier.

La règle d'arrêt est l'initiation d'une transfusion d'échange en accord avec les directives des pratiques cliniques de l'American Academy of Pediatrics Subcommitee on Hyperbilirubinemia (Pediatrics. 2004 Jul; 114(1) :297-316).

Le taux de bilirubine est mesuré, par un des nombreux tests connus, de manière à suivre l'évolution de la rémission du patient.

### Listes des références

[1] Cohn et al. 1946, J. Am. Chem. Soc. 68, 459 ; Oncley et al. 1949, J. Am. Chem. Soc. 71, 541.
[2] Gottstein et al. (2003), Archives of Disease in Childhood: Fetal and Neonatal Edition, vol. 88, no. 1, p. F6-F10.
[3] Alplay et al. (1999), Acta Paediatrica, International Journal of Paediatrics; vol. 88, no. 2, p. 216-219.
[4] Nasseri et al. (2006) Saudi Med J. ; 27(12) : 1827-30.
[5] Steinbuch et al. (1969) Rev. Franc. Et. Clin, et Biol., XIV, 1054.
[6] Buchta C. et al, Biologicals, 33, 2005, 41- 48.
[7] Wilson J. R. et al, Muscle & Nerve, 29(9), 1997, 1142- 1145.
[8] Copelan E.A et al, Transfusion, 26, 1986, 410-412.
[9] Misbah S.A et al, Drug Safety, 9, 1993, 254-262.
[10] Teeling J. L et al (Blood, 15/08/2001, vol. 98, number 4, pp.1095-1099.

## Revendications

1. Composition d'Immunoglobulines G (IgG) à usage thérapeutique, comprenant des titres respectifs en anticorps anti-A et anti-B conformes à un résultat négatif au test de Coombs indirect (Méthode 2.6.20 de la Pharmacopée européenne), pour son utilisation dans le traitement de l'ictère néonatal par incompatibilité foetomaternelle dans le système ABO ou de la maladie hémolytique du nouveau-né par incompatibilité ABO.

2. Composition pour l'utilisation selon la revendication 1, lesdits titres respectifs en anticorps anti-A et anti-B conformes à un résultat négatif au test de Coombs indirect étant de 64 en inverse de dilution.

3. Composition pour l'utilisation selon la revendication 1, lesdits titres respectifs en anticorps anti-A et anti-B conformes à un résultat négatif au test de Coombs indirect étant compris entre 0 et 8 en inverse de dilution.

4. Composition pour l'utilisation selon l'une quelconque des revendications précédentes, ledit test de Coombs indirect étant mis en oeuvre avec une solution d'IgG de concentration initiale ramenée à 30g/l.

5. Composition pour l'utilisation selon l'une quelconque des revendications précédentes, présentant une teneur d'IgG polyréactives résiduelle comprise entre 0,01% et 0,1%, par rapport à la teneur totale en IgG.

6. Composition pour l'utilisation selon l'une des revendications précédentes, comprenant en outre des stabilisants.

7. Composition pour l'utilisation selon l'une des revendications précédentes, dans lequel les stabilisants représentent un mélange d'un sucre alcoolique, de préférence du mannitol ou du sorbitol, de la glycine et d'un détergent non ionique.

8. Composition pour l'utilisation selon l'une des revendications précédentes, injectable par voie intraveineuse.

9. Composition pour l'utilisation selon l'une quelconque des revendications précédentes obtenu par un procédé comprenant les étapes suivantes :
a) préparation d'un concentré d'IgG par fractionnement éthanolique et/ou par séparation chromatographique, comprenant une étape d'inactivation virale,
b) chromatographie d'immunoaffinité par percolation dudit concentré d'IgG sur un mélange de supports dont les matrices sont greffées de groupes oligosaccharides antigéniquement similaires aux groupes sanguins A et B, et
c) filtration d'élimination de virus et/ou de particules de taille supérieure à 20 nm.

10. Composition pour l'utilisation selon la revendication 9, l'étape a) dudit procédé comprenant une prépurification par précipitation de contaminants lipidiques à partir d'un plasma sanguin ou d'une fraction de plasma sanguin enrichie en IgG, une chromatographie unique sur un support de résine échangeuse d'anions effectuée à pH alcalin et une élution sélective des IgG en une étape par un tampon approprié à pH compris entre 4 et 7.

11. Composition pour l'utilisation selon la revendication 9 ou 10, les groupes oligosaccharides dudit procédé représentant des trisaccharides correspondant aux épitopes des groupes sanguins A et B.

12. Composition pour l'utilisation selon l'une des revendications 8 à 11, l'étape d'inactivation virale dudit procédé étant effectuée par solvant-détergent.

13. Composition pour l'utilisation selon l'une des revendications 8 à 12, ledit mélange de supports greffés de groupes antigéniquement similaires au groupe sanguin A et au groupe sanguin B étant en une proportion respective comprise entre 25/75 et 75/25 (v/v), de préférence 50/50 (v/v) en chacun desdits supports.

14. Composition pour l'utilisation selon l'une des revendications 8 à 13, ledit procédé comprenant des étapes de concentration par ultrafiltration et de filtration stérilisante.

15. Composition pour l'utilisation selon la revendication 14, ladite étape de filtration stérilisante étant effectuée par nanofiltration.

16. Composition pour l'utilisation selon l'une des revendications 9 à 15, ledit procédé comprenant, après l'étape c), une étape d'ajout de stabilisants de conservation audit concentré d'IgG.

## Patentansprüche

1. Zusammensetzung aus Immunoglobulinen G (IgG) zur therapeutischen Verwendung, umfassend einen jeweiligen Anti-A-Antikörper- und Anti-B-Antikörpertiter in Übereinstimmung mit einem negativen Ergebnis im indirekten Coombs-Test (Methode 2.6.20 des Europäischen Arzneibuchs) zur Verwendung als Medikament zur Behandlung von Gelbsucht bei Neugeborenen durch Mutter-Kind-Inkompatibilität im ABO-System oder hämolytische Erkrankung des Neugeborenen durch ABO-Inkompatibilität.

2. Zusammensetzung zur Verwendung nach Anspruch 1, wobei der jeweilige Anti-A- und der Anti-B-Antikörpertiter in Übereinstimmung mit dem negativen Ergebnis im indirekten Coombs-Test bei 64 liegt umgekehrt zur Verdünnung,

3. Zusammensetzung zur Verwendung nach Anspruch 1, wobei der jeweilige Anti-A- und der Anti-B-Antikörpertiter in Übereinstimmung mit dem negativen Ergebnis im indirekten Coombs-Test zwischen 0 und 8 liegt umgekehrt zur Verdünnung.

4. Zusammensetzung zur Verwendung nach einem der vorhergehenden Ansprüche, wobei der indirekte Coombs-Test mit einer IgG Lösung durchgeführt wird, deren Ausgangskonzentration auf 30g/l rückgeführt wurde.

5. Zusammensetzung zur Verwendung nach einem der vorhergehenden Ansprüche, wobei der Gehalt an restlichen polyreaktiven IgG zwischen 0,01 % und 0,1% bezogen auf den IgG-Gesamtgehalt liegt.

6. Zusammensetzung nach einem der vorhergehenden Ansprüche ferner Stabilisatoren umfassend.

7. Zusammensetzung nach einem der vorhergehenden Ansprüche, wobei die Stabilisatoren eine Mischung aus einem Zuckeralkohol, vorzugsweise Mannitol oder Sorbitol, Glycin und einem nicht-ionischen Detergens darstellen.

8. Zusammensetzung zur Verwendung nach einem der vorhergehenden Ansprüche zur intravenösen Injektion.

9. Zusammensetzung zur Verwendung nach einem der vorhergehenden Ansprüche, erhältlich durch ein Verfahren umfassend die Schritte:
a) Herstellen eines IgG-Konzentrats durch Ethanol-Fraktionierung und / oder durch chromatographische Trennung, umfassend einen Schritt der Virusinaktivierung,
b) Immunaffinitätschromatographie durch Perkolation des IgG-Konzentrats auf eine Trägermischung deren Matrizen mit antigenisch den Blutgruppen A und B ähnlichen Oligosaccharid-Gruppen gepfropft sind, und
c) Filtration zum Abscheiden der Viren oder Partikel, die größer als 20 nm sind.

10. Zusammensetzung zur Verwendung nach Anspruch 9, wobei der Schritt a) des Verfahrens Folgendes umfasst : eine Vorreinigung durch Präzipitation von Lipid-Verunreinigungen aus einem Blutplasma oder einer mit IgG angereicherten Blutplasmafraktion, eine einzige bei alkalischem pH durchgeführte Chromatographie auf einem Anionentauscher-Harzträger und eine selektive Elution von IgG in einem Schritt mit einem geeigneten Puffer bei einem pH-Wert zwischen 4 und 7.

11. Zusammensetzung zur Verwendung nach Anspruch 9 oder 10, wobei die Oligosaccharid-Gruppen des Verfahrens Trisaccharide darstellen, die den Epitopen der Blutgruppen A und B entsprechen.

12. Zusammensetzung zur Verwendung nach einem der Ansprüche 8 bis 11, wobei der Schritt der Virusinaktivierung des Verfahrens durch Lösungsmittel-Detergens durchgeführt wird.

13. Zusammensetzung zur Verwendung nach einem der Ansprüche 8 bis 12, wobei die Mischung der mit der Blutgruppe A und der Blutgruppe B antigenisch ähnlichen Gruppen gepfropften Träger in einem jeweiligen Verhältnis von zwischen 25:75 und 75:25 (v / v) vorzugsweise 50:50 (v / v) in jedem der Träger ist.

14. Zusammensetzung zur Verwendung nach einem der Ansprüche 8 bis 13, wobei das Verfahren die Schritte der Konzentration durch Ultrafiltration und der Sterilfiltration umfasst.

15. Zusammensetzung zur Verwendung nach Anspruch 14, wobei der Schritt der Sterilfiltration durch Nanofiltration durchgeführt wird.

16. Zusammensetzung zur Verwendung nach einem der Ansprüche 9 bis 15, wobei das Verfahren nach Schritt c) einen Schritt der Zugabe von Lagerstabilisatoren zu dem IgG-Konzentrat umfasst.

## Claims

1. Immunoglobulin G (IgG) composition for therapeutic use, comprising respective anti-A and anti-B antibody titers in accordance with a negative result to the indirect Coombs test (method 2.6.20 of the European Pharmacopoeia), as a medicament for the treatment of neonatal jaundice caused by maternal-foetal incompatibility with respect to the ABO system or of neonatal haemolytic disease of the new-born caused by ABO incompatibility.

2. Composition for the use according to claim 1, said respective anti-A and anti-B antibody titers in accordance with a negative result to the indirect Coombs test being 64, reverse dilution.

3. Composition for the use according to claim 1, said respective anti-A and anti-B antibody titers in accordance with a negative result to the indirect Coombs test being between 0 and 8, reverse dilution.

4. Composition for the use according to any one of the preceding claims, said indirect Coombs test being carried out with an IgG solution with an initial concentration adjusted to 30 g/l.

5. Composition for the use according to any one of the preceding claims, having a residual polyreactive IgG content of between 0.01% and 0.1% with respect to the total IgG content.

6. Composition for the use according to one of the preceding claims, further comprising stabilizers.

7. Composition for the use according to one of the preceding claims, in which the stabilizers represent a mixture of an alcohol sugar, preferably mannitol or sorbitol, glycine and non-ionic detergent.

8. Composition for the use according to one of the preceding claims, injectable intravenously.

9. Composition for the use according to any one of the preceding claims, obtained by a method comprising the following steps:
a) preparation of an IgG concentrate by ethanol fractionation and/or by chromatographic separation, comprising a viral inactivation step,
b) immunoaffinity chromatography by percolation of said IgG concentrate on a mixture of carriers, the matrices of which are grafted with oligosaccharide groups antigenically similar to blood groups A and B, and
c) elimination filtration of viruses and/or particles with a size greater than 20 nm.

10. Composition for the use according to claim 9, step a) of said method comprising a prepurification by precipitation of lipid contaminants using a blood plasma or a fraction of blood plasma enriched with IgG, a single chromatography on an anion exchange resin carrier carried out at alkaline pH and a selective elution of the IgGs in one step using a suitable buffer with a pH of between 4 and 7.

11. Composition for the use according to claim 9 or 10, the oligosaccharide groups of said method representing trisaccharides corresponding to the epitopes of blood groups A and B.

12. Composition for the use according to one of claims 8 to 11, the viral inactivation step of said method being performed by solvent/detergent.

13. Composition for the use according to one of claims 8 to 12, said mixture of grafted carriers of groups antigenically similar to blood group A and to blood group B being in respective proportion of between 25/75 and 75/25 (v/v), preferably 50/50 (v/v) in each of said carriers.

14. Composition for the use according to one of claims 8 to 13, said method comprising steps of concentration by ultrafiltration and sterilising filtration.

15. Composition for the use according to claim 14, said sterilising filtration step being performed by nanofiltration.

16. Composition for the use according to one of claims 9 to 15, said method comprising, after step c), a step of adding preservation stabilizers to said IgG concentrate.
